# EUROPEAN PATENT APPLICATION

(11) **EP 3 848 940 A1**
(43) Date of publication of application: **14.07.2021**
(21) Application number: 20150644.1
(22) Date of filing: 08.01.2020
(51) Int. Cl.: G16H 30/40

(54) **A SYSTEM AND METHOD FOR TRIGGERING AN ACTION BASED ON A DISEASE SEVERITY OR AFFECTIVE STATE OF A SUBJECT**

(71) Applicant: Koninklijke Philips N.V., 5656 AG Eindhoven (NL)
(72) Inventor: DELLIMORE, Kiran Hamilton J., 5656 AE Eindhoven (NL); SAINI, Privender Kaur, 5656 AE Eindhoven (NL); PRIORI, Rita, 5656 AE Eindhoven (NL); KARAMOLEGKOS, Nikolaos, 5656 AE Eindhoven (NL)
(74) Representative: Philips Intellectual Property & Standards

(57) **Abstract**

A system is provided for triggering an action based on a disease severity and/or an affective state of a subject. Sensor data are used which monitors physiological conditions of the subject as well as user data which convey a subjective assessment of their affective state. An objective health indication is obtained from the sensor data and a subjective health indication is obtained from the user data. These indications are compared and this comparison is used in estimating a disease severity. A trigger for action by the subject or by a caregiver is generated based on the estimated disease severity and/or affective state.

## Description

### FIELD OF THE INVENTION

The invention relates to a system and method for triggering an action based on a disease severity of a subject or an effective state of a subject. In particular, it relates to a home monitoring system and method, wherein the triggered action may relate to the need for medical intervention.

### BACKGROUND OF THE INVENTION

Millions of patients with chronic lung diseases are now living at home, which means that they are monitored infrequently and necessary interventions are often significantly delayed. This has led to a sharp rise in costly emergency visits due to exacerbation of the patient condition, typically resulting in hospital readmission.

Home monitoring of patients with unobtrusive sensors is technically feasible, however, the large volume of data generated creates a new burden on healthcare providers in terms of effective data management and correct interpretation of the information carried by the large number of parameters being measured. Remote patient monitoring relies on a large number and variety of measures, both objective and subjective.

Objective measures are typically collected by specific sensors, either individually or allowing multiple data points to be collected by the same sensor or device.

Subjective measures are typically based on collecting self-reported information from individuals, and the information relates to their own perception and experience of any aspects related to a particular topic being investigated.

It has been recognized that both objective and subjective information is of interest for assessing a patient condition. For example, EP 1 911 396 discloses a home tele monitoring system for chronic disease in which subjective and objective data are processed. However, the interpretation of the information remains the task of a professional care provider.

There remains a need to be able to interpret this data in order to reliably reach suitable conclusions in an automated (computer-implemented) way.

### SUMMARY OF THE INVENTION

The invention is defined by the claims.

According to examples in accordance with an aspect of the invention, there is provided a system for triggering an action based on a disease severity or affective state of a subject, comprising:
a set of sensors providing sensor data for monitoring physiological conditions of the subject;
an input for receiving user data from the subject which convey a subjective assessment of their affective state;
a processor which is adapted to:
   derive from the sensor data an objective health indication;
   derive from the user data a subjective health indication;
   compare the objective and subjective health information;
   estimate a disease severity and/or an affective state of the subject based on the sensor data, the user data and the comparison of the objective and subjective health information; and
   provide a trigger for action by the subject or by a caregiver based on the estimated disease severity and/or affective state.

This system provides an estimate of a disease severity and/or an effective state, for a subject (i.e. patient) being monitored with a particular disease, typically in a home monitoring system. The system is typically for collecting large volumes of monitoring data obtained from a chronic disease patient in a domestic setting. The system aims to address the issue that the monitoring data do not present specific, clinically actionable and useful information due to the large volume of data. Sensor data alone also present a lack of subject-specific insights, in particular related to the affective state (e.g. mood) of the subject. The invention provides a system in which this affective state can be used to tailor and enhance the interpretation of data and enhance triggered actions, such as clinical interventions.

To achieve this, objective monitoring data are compared with subjective monitoring data. The objective monitoring data include at least some physiological information obtained from sensors such as unobtrusive monitoring sensors, but optionally may include other types of information from other sources including therapeutic devices, medical databases, non-clinical contextual databases such as weather forecasts, GPS location, etc. The subjective user data are for example obtained from subject input via a questionnaire, messaging, texting, surveys, telephone call, etc. The comparison provides additional subject-specific insights, in particular related to their affective state. These insights are used to trigger enhanced and tailored actions such as clinical interventions.

In particular, the comparison of subjective and objective health indications enables more reliable determination of both the state of disease progression as well as the affective state of the subject.

The trigger for action may be to trigger one or more of:
an alert or warning;
an indication that clinical intervention is needed;
a request from the subject for additional user data;
the collection of additional sensor data;
adjustment of a therapy device setting;
adjustment of a sensor setting;
display of advisory information or motivational information to the subject.

Thus, various actions may be triggered. If an emergency is considered, there will be an indication that clinical intervention is needed. This will for example be communicated automatically to a remote care provider system. Indeed, all the collected data will be provided to a remote central system with which the home-based system communicates.

The objective health indication and the subjective health indication may each comprise a numerical value on the same numerical scale, and the comparison comprises a difference or ratio between the numerical values. The subjective and objective health indications are thus generated in a form which enables a comparison between numerical values. The numerical scale may for example be a value from 0 to 10 or an integer from 0 or 1 to 10.

The processor may be adapted to estimate the disease severity and/or the affective state from:
the sensor data if the comparison falls within a first range; or
trends in the sensor data and in the user data if the comparison falls within a second range.

If the comparison is within a first range, indicating a close correlation between the objective and subjective health indications, the sensor data may be used. This is beneficial because more regular and a greater volume of sensor data are typically available.

If the comparison is within a second range, indicating a poor correlation between the objective and subjective health indications, trends in both data types may be used. The first and second ranges may together encompass all possible values.

The processor may be further adapted to determine an external influence to the affective state of the subject, for example based on the objective health indication improving and the subjective health indication deteriorating. The external influence may be the cause of a change in the affective state which is not related to their medical condition but may still warrant a trigger.

The processor may be further adapted to determine from the sensor data and the user data whether sufficient data are available to estimate a disease severity and/or an affective state, and if not:
to collect additional sensor data and/or user data; or
to perform data imputation.

This provides an initial data screening step to ensure the estimate of disease severity and/or the affective state is sufficiently reliable. This ensures adequate subjective and objective data are provided to enhance the effectiveness of any intervention.

The system may further comprise an activity monitor for monitoring activity of the subject, and the processor is adapted to provide a trigger requiring action by the subject at a time dependent on the activity monitoring.

This enables the receptiveness of the subject to the trigger to be taken into account, to improve the likelihood of the necessary action being taken. The activity monitor may for example determine if the subject is at rest, walking or driving, and the affective state can also be taken into account to determine the likely receptiveness of the subject to the requested action.

The processor may be further adapted to:
receive user input in response to a triggered action which comprises a medical intervention; and
adapt the triggering of future such medical interventions based on the received user input.

This provides a self-learning function by which the effectiveness of interventions can be improved over time.

The invention also provides a computer-implemented method for triggering an action based on a disease severity and/or an affective state of a subject, comprising:
receiving sensor data in respect of physiological conditions of the subject;
receiving user data from the subject which convey a subjective assessment of their affective state;
deriving from the sensor data an objective health indication;
deriving from the user data a subjective health indication;
comparing the objective and subjective health information;
estimating a disease severity and/or an affective state based on the sensor data, the user data and the comparison of the objective and subjective health information; and
providing a trigger for action by the subject or by a caregiver based on the estimated disease severity.

This is the method implemented by the system above. The estimate of disease severity and/or the affective state is not a clinical diagnosis but is an indicator for determining whether clinical intervention or other actions are required.

As mentioned above, the method may comprise triggering one or more of:
an alert or warning;
an indication that clinical intervention is needed;
a request from the subject for additional user data;
the collection of additional sensor data;
adjustment of a therapy device setting;
adjustment of a sensor setting;
display of advisory information or motivational information to the subject.

The disease severity and/or the affective state may be estimated from:
the sensor data if the comparison falls within a first range; or
trends in the sensor data and in the user data if the comparison falls within a second range.

The method may also comprise determining from the sensor data and the user data whether sufficient data are available to estimate a disease severity and/or an affective state, and if not:
collecting additional sensor data and/or user data; or
perform data imputation.

The method may further comprise monitoring activity of the subject, and providing a trigger requiring action by the subject at a time dependent on the activity monitoring.

The method may also comprise:
receiving user input in response to a triggered action which comprises a medical intervention; and
adapting the triggering of future such medical interventions based on the received user input.

The method may be implemented in software.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for triggering an action based on a disease severity and/or affective state of a subject;
Figure 2 shows a first example of the functions performed by the processor;
Figure 3 shows how the subject's affective state may be inferred from the health indicators provided by the processor of Figure 2;
Figure 4 shows a hierarchical decision tree for selecting possible output triggers;
Figure 5 shows a second example of the functions performed by the processor;
Figure 6 shows a third example of the functions performed by the processor;
Figure 7 shows a fourth example of the functions performed by the processor; and
Figure 8 shows a computer-implemented method for triggering an action based on a disease severity and/or the affective state of a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for triggering an action based on a disease severity and/or an affective state of a subject. Sensor data are used which monitor physiological conditions of the subject as well as user data which convey a subjective assessment of their affective state. An objective health indication is obtained from the sensor data and a subjective health indication is obtained from the user data. These indications are compared and this comparison is used in estimating a disease severity. A trigger for action by the subject or by a caregiver is generated based on the estimated disease severity and/or the affective state.

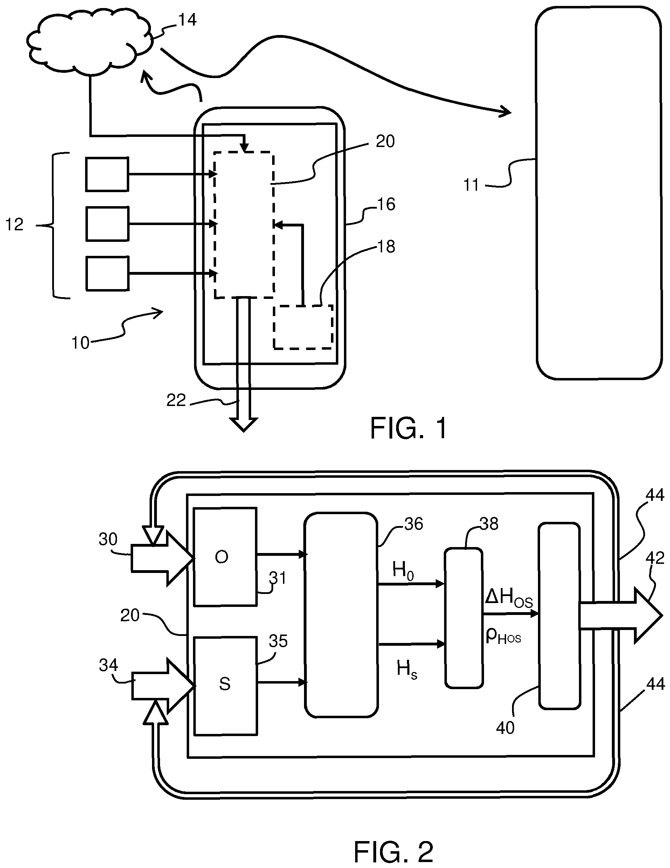

Figure 1 shows a system for triggering an action based on a disease severity and/or affective state of a subject.

The overall system comprises a user monitoring device 10 and a central control center 11.

The user monitoring device 10 is in particular for monitoring subjects (i.e. patients) with a chronic disease. Such diseases necessitate longitudinal monitoring (generating large volumes of subject data) and long term disease management. In particular, the user monitoring device is for self-management in a home setting. Many chronic disease patients lack motivation for self-care and are at an elevated risk of depression, which can have adverse effects on disease management and outcomes. The aim of the system is to determine the subject's affective (i.e., emotional) state so that clinical remote monitoring interventions, such as a chronic care program, can be enhanced and better tailored to the subject's needs at a given moment in time.

The central control center 11 is administered by a health care provider. It enables a remote health care provider to assess when the subject is in need of a medical intervention or assistance.

The user monitoring device 10 comprises a set 12 of sensors providing (at least) sensor data for monitoring at least physiological conditions of the subject. These sensor data are objective data, in that they provide a measurement of known parameters.

The set of sensors generates a set of N input data streams from one or more objective monitoring sources. The sensors include unobtrusive monitoring sensors for 24/7 monitoring of the subject, but there may be additional inputs other than from physiological sensors, such as feedback from therapeutic devices, (historical) medical databases, non-clinical contextual databases (e.g., weather forecasts, GPS location, etc.).

Figure 1 shows a data cloud 14 as an example of a source of external objective data, such as weather reports, pollution sensing, etc. The user monitoring device is shown as a mobile phone 16 in this example, and it may also have integrated sensors 18, such as an accelerometer, a gyroscope, etc.

The user monitoring device 10 also has a user input interface, such as a touch screen for the example of a mobile phone. This enables subjective user input. The subjective user input is for example subject input via a questionnaire, telephone call, messaging, texting, surveys or other means. The user data convey a subjective assessment of their affective state.

The processor 20 derives from the sensor data an objective health indication and also derives from the user data a subjective health indication. As will be explained in more detail below, the objective and subjective health indications are compared. A disease severity and/or an affective state is then estimated based on the sensor data, the user data and the comparison of the objective and subjective health indications. This then enables a trigger for action by the subject or by a caregiver to be derived based on the estimated disease severity.

This system for example provides an estimate of a disease severity, for a disease being monitored. The affective state of the subject is also determined, and is used to tailor and enhance the interpretation of data and enhance triggered actions, such as clinical interventions.

Figure 1 shows a trigger 22 for action by the subject or by a caregiver, provided as an output from the user monitoring device 10 to the subject and/or caregiver.

The signals measured by the sensors will vary from disease to disease, and may indeed be aggregated given that chronic disease patients often have multiple comorbidities. Examples are movement data, heart rate variability, SpO₂ measurements, body temperature measurements, etc.

The subjective user data are typically prompted input via questionnaires, messaging, texting, surveys, or phone calls, etc. The subjective data typically have a lower sampling frequency than the objective data.

The comparison of the objective and subjective health indications gives an insight into the subject's affective state, for example by considering the magnitude of the difference between health indications derived from objective and subjective data. Large mismatches between health indications derived from these two data sources are indicative of the subject's fluctuating affective state. For example, if the objective health indication indicates the subject's condition is stable or slightly improving over time, while the subjective health indication suggests that their health status is gradually worsening, this is likely indicative of a negative affective state.

The invention makes use of the correlation between affective state and physical health. Mood for instance influences health-relevant behaviors. Furthermore, positive emotions and healthy outcomes are likely to be linked through multiple psychological pathways.

The trigger 22 for action may be to trigger one or more of:
an alert or warning to the subject or to the caregiver;
an indication that clinical intervention is needed, either immediately or with a specified delay;
a request from the subject for additional (subjective) user data;
the collection of additional (objective) sensor data;
adjustment of a therapy device or monitoring device setting;
adjustment of a sensor setting;
display of advisory information or motivational information to the subject. This may be training regimes.

As an example, if a subject is a determined to be in a depressive affective state, clinical intervention may be triggered resulting in a call from the subject's doctor, or the intensity of the subject's exercise regime may be reduced for a given time period.

All the collected data and all the triggered actions will be provided to the remote central system 11.

Figure 2 shows a first example of the functions performed by the processor 20.

Sensor outputs are received as an input 30 to the processor, and from this objective data 31 are generated. User outputs are received as input 34 and from this user data 35 are generated which convey a subjective assessment of the affective state of the user.

The processor comprises a health status sub-unit 36, a data comparison sub-unit 38 and a triggering sub-unit 40. The processor processes a set of parallel input data streams. As explained above, the objective data are derived from sensors such as unobtrusive monitoring sensors, but also from other devices and information sources, such as therapeutic devices, (historical) medical databases, non-clinical contextual databases (e.g., weather forecasts, GPS location, etc.).

The health status sub-unit 36 generates an objective health indication Hₒ for the subject based on the objective data. This will be termed an objective health status score. The health status sub-unit 36 also generates a subjective health indication Hₛ for the subject based on the subjective user data input. This will be termed a subjective health status score.

Hₒ and Hₛ are for example scalar numbers ranging from 1-10 or a similar scale.

The data comparison sub-unit 38 finds the difference, ΔHₒₛ, between Hₒ and Hₛ or alternatively, the ratio of Hₒ to Hₛ may also be computed, ρ_{Hos} = Hₒ/Hₛ. Insight into the subject's affective state is obtained by the data comparison sub-unit 38 by considering the magnitude of ΔHₒₛ or ρ_{Hos}.

If the difference between Hₒ and Hₛ is small (e.g., |ΔHₒₛ| ≤ 15% or 0.85 ≤ ρ_{Hos} ≤ 1.15) then the subject's affective state is inferred from the trend in the objective data. The trend in the objective data is preferably used since the objective data volume and frequency are typically higher than the volume and frequency of the subjective data. Subjective data input typically may be requested 2-4 times per week to minimize burden on the subject being monitored, while the objective data input may be obtained at regular intervals at a rate of the order of a few hours. For example, if the objective data (recorded e.g. every 6 hours) indicate a downward trend in health status (over for example 3 days), then the subject's affective state can be inferred to be at risk of being more depressive or adverse. Conversely, if the objective data indicate an upward trend in health status, then there is higher likelihood that the subject's affective state can be inferred to be happier or more positive/optimistic.

If the difference between Hₒ and Hₛ is large (e.g., |ΔHₒₛ|> 15% or ρ_{Hos} < 0.85 or ρ_{Hos} > 1.15) then the subject's affective state is inferred from the trends in both the objective and subjective data. In addition, it can be further inferred that an external unknown factor beyond the disease condition of the subject may be influencing the subject's affective state. For example, if the trend in the objective data indicates that the subject's health status is stable or improving, yet their subjective data indicate that their health status is declining, it can be inferred that the subject is in an adverse affective state. Conversely, if the trend in the objective data indicates that the subject's health status is unstable or declining, yet their subjective data indicate that their health status is stable or improving, it can be inferred that the subject is in a positive or neutral affective state.

Furthermore, the larger the magnitude of the difference between Hₒ and Hₛ, the stronger the assumed influence of the subject's affective state.

Thus, the processor may estimate one or both of a disease severity and an affective state from the sensor data if the comparison falls within a first range, or from trends in the sensor data and in the user data if the comparison falls within a second range (e.g. outside the first range).

The triggering sub-unit 40 estimates a disease severity or an affective state or both based on the sensor data, the user data and the comparison of the objective and subjective health information. In response, it provides a trigger 42, which may relate to a set of possible actions, for action by the subject or by a caregiver based on the estimated disease severity or affective state. Trends in the disease severity and/or affective state are also identified.

The trigger 42 is fed back as input data by the feedback paths 44 so that the processor can take account of historical information in its decision making.

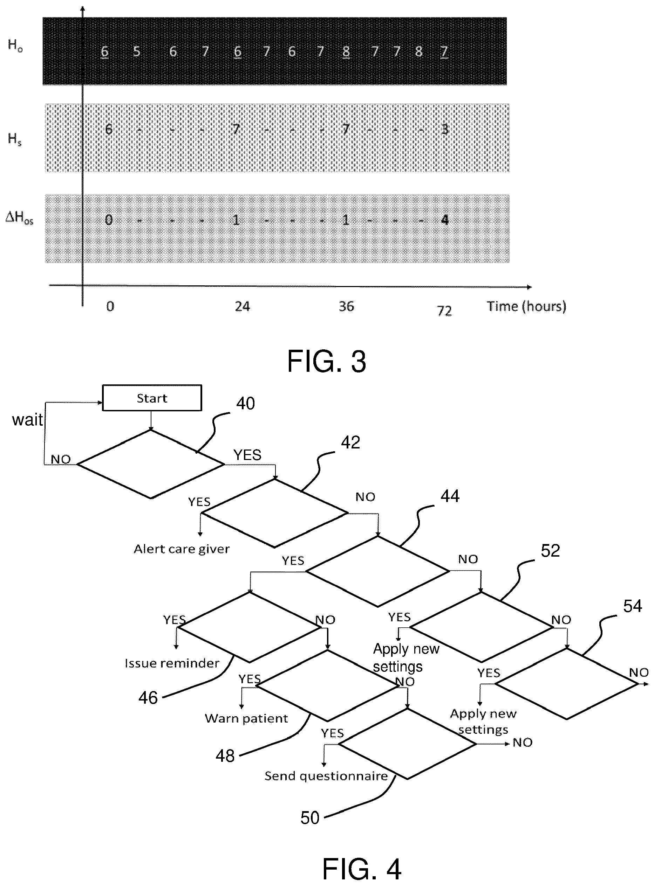

Figure 3 shows how the subject's affective state may be inferred from Hₒ and Hₛ and the comparison ΔHₒₛ. Figure 3 shows more frequent calculation of the objective health status score Hₒ than the calculation of the subjective health status score Hₛ. At each new value of Hₛ the comparison is made.

At the last calculation point in Figure 3 |ΔHₒₛ|> 50%, with Hₛ << Hₒ. This indicates that the subject is likely to have a strong negative or depressive affective state. In medicine it is assumed that subject perception outweighs objective data and is therefore a very important indicator of subject status.

The triggering sub-unit 40 is activated after the affective state of the subject has been inferred by the data comparison sub-unit 38. Depending on the determined affective state one or more of a set of outputs may be triggered. These outputs may include: alerts or warnings to the subjects and/or caregiver(s); immediate or delayed/planned clinical intervention' adjustment of monitoring or therapy device settings; motivational messages; (adapted) training regimes; reminders or requests for additional subjective input from the subject or additional objective data from the monitoring/therapy devices.

This may ideally be accomplished via a hierarchical decision tree as illustrated below in Figure 4.

In step 40, the need for an output trigger is determined.

In step 42, there is determination of whether a caregiver should be alerted.

If not, in step 44, there is determination of whether the subject should be alerted. There are three possible subject alerts shown, namely a reminder in step 46, or a warning in step 48 (e.g. to stop a current activity) or a request for additional subjective input in step 50.

In step 52, there is determination of whether monitoring sensor settings should be adjusted, such as a measurement frequency.

In step 54, there is determination of whether therapy device settings should be adjusted.

As an example, if a subject is a determined to be in a depressive affective state clinical intervention may be triggered resulting in a call from the subject's doctor, or the intensity of the subject's exercise regime may be reduced.

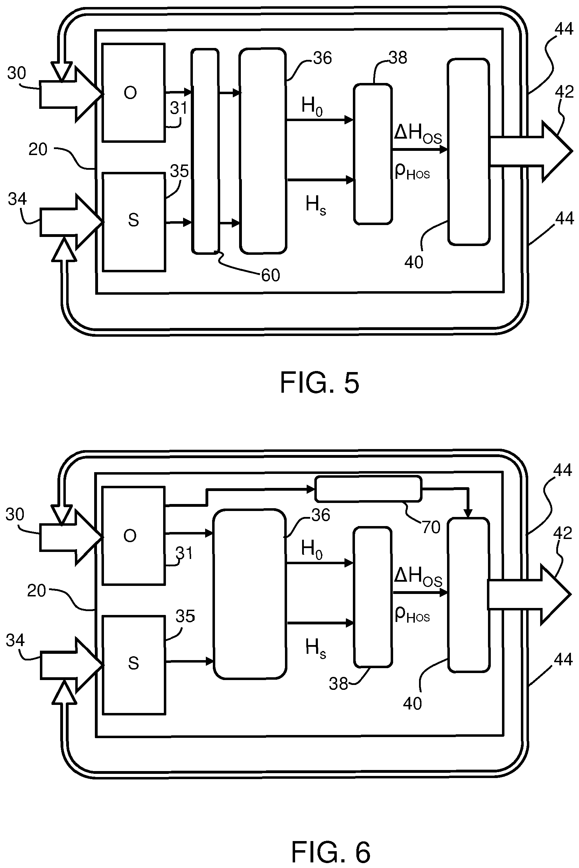

Figure 5 shows a second example which includes a data check sub-unit 60 to ensure that adequate objective and subjective data streams are available. The data check sub-unit 60 analyzes the objective and subjective data to determine if they are adequate both in quantity and quality/completeness in order for the health status sub-unit 36 to obtain a reliable estimate of the subject's health status.

If inadequate objective and/or subjective data are present the trigger unit 40 may be activated to request additional data input from the subject, or from monitoring or therapy devices. If not enough data are present, the data check-sub unit 60 may for example implement data imputation techniques on trend data in order to provide the health status sub-unit 36 with an estimate of the current data.

The adequacy of the data may be determined by the data-check sub-unit based on at least one of the following parameters: i) data quantity, ii) completeness of the data and iii) the quality/reliability of the data (e.g., excessive noise or data with artefacts may be discarded).

Data imputation in the case of missing data may be implemented by interpolation techniques for example if the above parameters can be satisfied based on historical trend data.

Figure 6 shows a third example which includes an activity classification sub-unit 70 for enhancing a clinical intervention based on the current activity state of the subject. The activity classification sub-unit 70 utilizes non-clinical contextual objective data, obtained from various sources such as devices either worn by the subject or in their vicinity, to determine the current activity state of the subject in order to enable more optimal timing for the delivery of outputs to the subject.

An activity monitor is used for monitoring activity of the subject (e.g. the accelerometer shown in Figure 1), and the processor then provides a trigger requiring action by the subject at a time dependent on the activity monitoring.

Depending on the activity being performed and the determined affective state of the subject, the trigger sub-unit 40 may deliver outputs immediately to the subject or after some finite time delay, t, at a more suitable moment, in order to enhance the receptiveness of the subject to the delivered output. For instance, if the subject is determined to be in a happy affective state and at rest then a reminder message to perform a self-care action involving more intense physical training will be better received than if the subject is in a depressive affective state, or while performing another task such as driving.

In latter case, the trigger system may delay delivery of the reminder until after the activity is completed or may deliver the reminder after requesting additional subjective subject input to determine that the subject's affective state has changed (i.e., improved).

Checking the affective state after completion of an activity could be used as additional feedback information, with the aim of increasing the likelihood that an activity will be performed in the future even when the affective state of the subject is less than optimal.

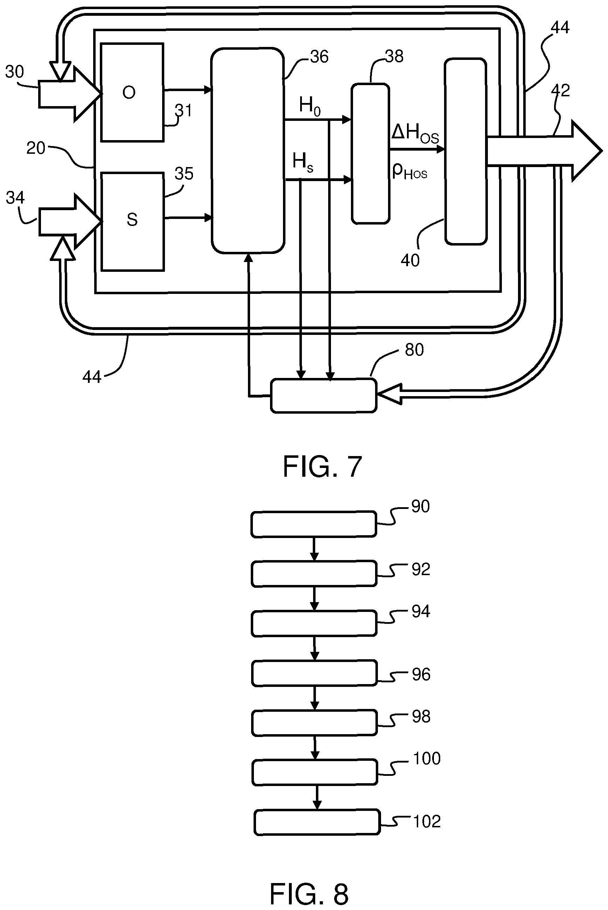

Figure 7 shows a fourth example which includes a learning sub-unit 80 for enhancing clinical intervention by learning from previous events. The learning implemented by the sub-unit 80 is based on requesting the user (subject or caregiver) to provide feedback on the effectiveness and appropriateness of the system's suggested interventions. Feedback can include, but is not limited to, a simple question (e.g., a rating from 0 to 10) or a more involved inquiry via a questionnaire, if evaluation of different aspects (effectiveness, appropriateness) of the intervention is desired.

For the learning process, the learning sub-unit for example continuously receives and stores (i) the health status scores (Hₒ and Hₛ) from the health status sub-unit 36, and (ii) the outputs generated by the triggering sub-unit 40, by means of the feedback paths 44. Once the system suggests an intervention, feedback from the user is requested. The user feedback is then utilized by the learning sub-unit 80 to validate and correct, if necessary, the logic of the health status and triggering sub-units. For example, if the user submits a low rating (indicating that the intervention was not effective with respect to the subject's actual condition) and the health status has been assessed as unstable or declining, then the learning sub-unit 80 may then take corrective actions to improve the health status assessment so that at the next triggering event, the suggested intervention is more effective.

Alternatively, the learning sub-unit 80 may also indirectly assess whether the triggered interventions are effective based on post-intervention changes in the trends of Hₒ and Hₛ. If the trends of both Hₒ and Hₛ show improvement, this may indicate the intervention(s) have been effective, while if both show a declining trend or are unchanged this may indicate less effective intervention. This approach may of course be used in combination with periodic user feedback to enhance clinical intervention.

Figure 8 shows a computer-implemented method for triggering an action based on a disease severity and/or the affective state of a subject. The method comprises:
in step 90, receiving sensor data in respect of physiological conditions of the subj ect;
in step 92, receiving user data from the subject which convey a subjective assessment of their affective state;
in step 94, deriving from the sensor data an objective health indication; and
in step 96, deriving from the user data a subjective health indication.

The objective and subjective health information are compared in step 98 so that a disease severity and/or an affective state may be estimated in step 100 based on the sensor data, the user data and the comparison of the objective and subjective health information. A trigger for action by the subject or by a caregiver is provided in step 102 based on the estimated disease severity and/or the affective state.

The skilled person would be readily capable of developing a processor for carrying out any herein described method. Thus, each step of a flow chart may represent a different action performed by a processor and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for triggering an action based on a disease severity and/or affective state of a subject, comprising:
a set of sensors (12,18) providing sensor data (31) for monitoring physiological conditions of the subject;
an input (34) for receiving user data (35) from the subject which convey a subjective assessment of their affective state;
a processor (20) which is adapted to:
derive from the sensor data (31) an objective health indication (Hₒ);
derive from the user data (35) a subjective health indication (Hₛ);
compare the objective and subjective health information;
estimate a disease severity and/or an affective state of the subject based on the sensor data, the user data and the comparison of the objective and subjective health information; and
provide a trigger (22) for action by the subject or by a caregiver based on the estimated disease severity and/or affective state.

2. A system as claimed in claim 1, wherein the trigger (22) for action is to trigger one or more of:
an alert or warning;
an indication that clinical intervention is needed;
a request from the subject for additional user data;
the collection of additional sensor data;
adjustment of a therapy device setting;
adjustment of a sensor setting;
display of advisory information or motivational information to the subject.

3. A system as claimed in claim 1 or 2, wherein the objective health indication (Hₒ) and the subjective health indication (Hₛ) each comprise a numerical value on the same numerical scale, and the comparison comprises a difference or ratio between the numerical values.

4. A system as claimed in claim 3, wherein the processor (20) is adapted to estimate the disease severity and/or the affective state from:
the sensor data (31) if the comparison falls within a first range; or
trends in the sensor data (31) and in the user data (35) if the comparison falls within a second range.

5. A system as claimed in any one of claims 1 to 4, wherein the processor (20) is further adapted to determine an external influence to the affective state of the subject, for example based on the objective health indication improving and the subjective health indication deteriorating.

6. A system as claimed in any one of claims 1 to 5, wherein the processor (20) is further adapted to determine from the sensor data and the user data whether sufficient data are available to estimate a disease severity and/or an affective state, and if not:
to collect additional sensor data and/or user data; or
to perform data imputation.

7. A system as claimed in any one of claims 1 to 6, wherein the set of sensors further comprise an activity monitor (18) for monitoring activity of the subject, and the processor (20) is adapted to provide a trigger requiring action by the subject at a time dependent on the activity monitoring.

8. A system as claimed in any one of claims 1 to 7, wherein the processor (20) is further adapted to:
receive user input in response to a triggered action which comprises a medical intervention; and
adapt the triggering of future such medical interventions based on the received user input.

9. A computer-implemented method for triggering an action based on a disease severity and/or the affective state of a subject, comprising:
(90) receiving sensor data (31) in respect of physiological conditions of the subj ect;
(92) receiving user data (35) from the subject which convey a subjective assessment of their affective state;
(94) deriving from the sensor data an objective health indication;
(96) deriving from the user data a subjective health indication;
(98) comparing the objective and subjective health information;
(100) estimating a disease severity and/or an affective state based on the sensor data, the user data and the comparison of the objective and subjective health information; and
(102) providing a trigger for action by the subject or by a caregiver based on the estimated disease severity and/or the affective state.

10. A method as claimed in claim 9, comprising triggering one or more of:
an alert or warning;
an indication that clinical intervention is needed;
a request from the subject for additional user data;
the collection of additional sensor data;
adjustment of a therapy device setting;
adjustment of a sensor setting;
display of advisory information or motivational information to the subject.

11. A method as claimed in claim 9 or 10, comprising estimating the disease severity and/or the affective state from:
the sensor data (31) if the comparison falls within a first range; or
trends in the sensor data (31) and in the user data (35) if the comparison falls within a second range.

12. A method as claimed in any one of claims 9 to 11, comprising determining from the sensor data and the user data whether sufficient data are available to estimate a disease severity and/or the affective state, and if not:
collecting additional sensor data and/or user data; or
perform data imputation.

13. A method as claimed in any one of claims 9 to 12, further comprising monitoring activity of the subject, and providing a trigger requiring action by the subject at a time dependent on the activity monitoring.

14. A method as claimed in any one of claims 9 to 13, comprising:
receiving user input in response to a triggered action which comprises a medical intervention; and
adapting the triggering of future such medical interventions based on the received user input.

15. A computer program comprising computer program code means which is adapted, when said program is run on a computer, to implement the method of any one of claims 10 to 14.
